# EUROPEAN PATENT APPLICATION

(11) **EP 4 193 940 A1**
(43) Date of publication of application: **14.06.2023**
(21) Application number: 21863076.2
(22) Date of filing: 26.10.2021
(51) Int. Cl.: A61B 17/34, A61B 34/32, A61B 17/00, A61B 34/30

(54) **MOUNTING MODULE FOR MOUNTING MULTI-CHANNEL HAIR-TRANSPLANTER AND AUTOMATIC HAIR-TRANSPLANTING DEVICE HAVING SAME**

(30) Priority: 13.10.2021 KR 20210135972
(71) Applicant: OHDAE Corporation, Dalseong-gun, Daegu 42984 (KR)
(72) Inventor: KIM, Byoung Sul, Yeongyang-gun, Gyeongsangbuk-do 36517 (KR); KIM, Jong Hoon, Daegu 41464 (KR); EOM, Ki Hyeon, Dalseong-gun, Daegu 42968 (KR)
(74) Representative: Grünecker Patent- und Rechtsanwälte PartG mbB
(86) International application number: PCT/KR2021/015094
(87) International publication number: WO 2023/063465

(57) **Abstract**

The present disclosure relates to a hair transplanter mounting module which is mounted on a robot arm configured with a plurality of arms and a plurality of joints, and an automatic hair transplant device having the same, and in an embodiment, the hair transplanter mounting module includes: a bracket which is coupled to the robot arm; a first sliding block which is installed on the bracket and is slidable in a vertical direction with respect to the bracket; and a second sliding block which is installed on the bracket and is disposed at one side of the first sliding block in parallel, and is slidable in the vertical direction with respect to the bracket, and a multi-channel hair transplanter module is removably mounted on the first sliding block 130.

## Description

### [Technical Field]

The present disclosure relates to a hair transplanter, and more particularly, to an automatic hair transplant device which is capable of automatically transplanting hair into patient's scalp by using a hair transplanter.

### [Background Art]

The hair transplant is one of hair therapy procedures that transplants hair by harvesting hair from a hair growth area of a scalp and implanting the hair into bald areas of the scalp (hair loss patient), and enables a hair loss patient to have rich hair like normal people.

A plurality of hair transplanters may be used for the hair transplant procedure. The hair transplanter is a device that transplants hair by harvesting hair from a hair growth area of a scalp and implanting the hair into a bald area of the scape (hair loss patient). A related-art hair transplanter may include one transplantation needle in one body, and may include a core shaft slidably disposed in the transplantation needle.

When the related-art hair transplanter is used, an operator grips the hair transplanter with his/her hand and operates the transplantation needle and the core shaft by manipulating (pressing or pulling up) one by one with his/her finger.

However, such a related-art single-shot passive type of hair transplanter only transplants one hair root with one hair transplanter. Therefore, in order to transplant many hair roots, a plurality of hair transplanters should be prepared and should be changed one by one every time an operation is performed, and also, operations by operator's finger required for operating the transplantation needle and the core shaft may be difficult to perform.

That is, in the case of the related-art hair transplanter, when an operator (for example, a surgeon), who performs the hair transplant procedure, finishes one operation of sticking a transplantation needle into a scalp and operating a core shaft, the operator should place the already used hair transplanter on a separate worktable, and then, should hold another prepared hair transplanter and repeat the same process on patient's scalp again. Since a normal hair transplant procedure is performed by 1000 or more transplanting operations for about 3-5 hours, the operator should manipulate the transplantation needle and the core shaft with his/her finger every time the transplanting operation is performed, and much labor is required to perform the hair transplant, and a high degree of fatigue is caused.

### [Prior Art Literatures]

### [Patent Literatures]

(Patent Literature 1) 1. Korean Patent Publication No. 2008-0049793 (published on June 4, 2008)
(Patent Literature 2) 2. Korean Patent Publication No. 2019-0109321 (published on September 25, 2019)

### [Detailed Description of the Present disclosure]

### [Technical Objects]

The present disclosure has been developed in order to solve the problems of the related-art technology described above, and an object of the present disclosure is to provide an automatic hair transplant device which is capable of automatically performing almost all processes of a hair transplanting operation, by automatically mounting a multi-channel hair transplanter provided with a plurality of needles (transplantation needles), transplanting hair into patient's scalp, and automatically placing on a stand.

### [Technical Solving Means]

According to an embodiment of the present disclosure, there are provided a hair transplanter mounting module which is mounted on a robot arm configured with a plurality of arms and a plurality of joints, and an automatic hair transplant device having the same, wherein the hair transplanter mounting module includes: a bracket which is coupled to the robot arm; a first sliding block which is installed on the bracket and is slidable in a vertical direction with respect to the bracket; and a second sliding block which is installed on the bracket and is disposed at one side of the first sliding block in parallel, and is slidable in the vertical direction with respect to the bracket, wherein a multi-channel hair transplanter module is removably mounted on the first sliding block 130.

In an embodiment, the hair transplanter mounting module may include a first rail which is formed on the bracket by a predetermined length in the vertical direction, and a second rail which is formed on the first sliding block by a predetermined length in the vertical direction, and is engaged with and slidably coupled with the first rail.

In an embodiment, the multi-channel hair transplanter module may include: a multi-channel hair transplanter provided with a plurality of needles which are arranged around a center axis in a radial direction; and a hair transplanter holder which is removably coupled to a sliding handle on an outer circumference of the multi-channel hair transplanter.

In an embodiment, a third rail may be formed on the first sliding block by a predetermined length in the vertical direction, and the hair transplanter holder may include a fourth rail which is formed on one side surface of the hair transplanter holder and is engaged with the third rail and is coupled to be slidable in the vertical direction.

In an embodiment, the first sliding block may further include a holder locking protrusion formed on a surface of the first sliding block that faces the hair transplanter holder, and the hair transplanter holder may further include a first recess which is formed on a surface of the hair transplanter holder that faces the first sliding block, and accommodates the holder locking protrusion. In addition, the holder locking protrusion is inserted into the first recess with the third rail of the first sliding block and the fourth rail of the hair transplanter holder being engaged with each other, such that the multi-channel hair transplanter module is mounted on the first sliding block.

In an embodiment, the hair transplanter mounting module may further include a bush bar which is installed on an upper portion of the third rail of the first sliding block and is configured to press down the hair transplanter holder mounted on the first sliding block by using an elastic force.

In an embodiment, when the multi-channel hair transplanter module is mounted on the first sliding block, the second sliding block may be configured to press an upper end of the multi-channel hair transplanter module.

According to an embodiment of the present disclosure, there is provided an automatic hair transplant device which automatically performs a hair transplant operation of transplanting hair into patient's scalp by using a hair transplanter, the automatic hair transplant device including: a hair transplanter mounting module attached to a multi-joint robot arm; one or more stands configured to hold a plurality of multi-channel hair transplanter modules; and a controller configured to control operations of the robot arm and the hair transplanter mounting module, wherein the robot arm and the hair transplanter mounting module are operated under control of the controller to perform an operation of mounting the multi-channel hair transplanter module held on the stand on the hair transplanter mounting module, and an operation of placing the multi-channel hair transplanter module mounted on the hair transplanter mounting module on the stand.

In an embodiment, the stand may include a plurality of hair transplanter seating portions configured to accommodate the multi-channel hair transplanter modules, respectively, and a lever installed adjacent to the hair transplanter seating portions, and, when the hair transplanter mounting module having the multi-channel hair transplanter module mounted thereon moves down, the hair transplanter mounting module presses one end of the lever and the multi-channel hair transplanter module is dismounted from the first sliding block by the lever.

In an embodiment, the one or more stands may include a first stand and a second stand, and the first stand may hold one or more multi-channel hair transplanter modules before the multi-channel hair transplanter modules are used for a hair transplant procedure, and the second stand may hold one or more multi-channel hair transplanter modules which are used for the hair transplant procedure.

### [Advantageous Effects]

According to an embodiment of the present disclosure, the hair transplanter mounting module individually controls and performs operations of gripping a mounted hair transplanter and pressing an upper cap, and accordingly, a hair transplant operation which is performed by an operator holding a hair transplanter may be automatically performed.

In addition, according to an embodiment of the present disclosure, the automatic hair transplant device may automatically perform an operation of mounting the hair transplanter on the hair transplanter mounting module, an operation of transplanting air into patient's scalp by using the mounted hair transplanter, and an operation of holding the mounted hair transplanter on the stand.

### [Brief Description of the Drawings]

FIG. 1 is a perspective view of an automatic hair transplant device using a multi-channel hair transplanter according to an embodiment of the present disclosure;
FIG. 2 is a view provided to explain the multi-channel hair transplanter and a holder supporting the same according to an embodiment;
FIGS. 3 and 4 are perspective views of a hair transplanter mounting module having the multi-channel hair transplanter mounted thereon;
FIGS. 5 and 6 are perspective views of the hair transplanter mounting module when the multi-channel hair transplanter is not mounted;
FIG. 7 is a perspective view of a hair transplanter stand according to an embodiment;
FIG. 8 is a view provided to explain an operation of mounting a hair transplanter module on the hair transplanter mounting module;
FIG. 9 is a view provided to explain an operation of placing the hair transplanter module mounted on the mounting module on the stand;
FIG. 10 is a view illustrating an exemplary configuration of an entire automatic hair transplant device system provided with a robot arm and a hair transplanter mounting module according to an embodiment;
FIGS. 11 and 12 are enlarged views of the hair transplanter mounting module mounted in an end of the robot arm; and
FIG. 13 is a view provided to explain devices arranged in a main body of the system.

### [Explanation of Signs]

10: hair transplanter mounting module 20: hair transplanter module
30: multi-channel hair transplanter 40: hair transplanter holder
50: robot arm 60, 60': hair transplanter stand
70: 3D scanner 80: keyboard
90: display 100: bracket
110, 120: driving unit 130, 140: sliding block
150: rotation member 160: push bar
200: system main body

### [Best Mode for Embodying the Invention]

Exemplary embodiments will now be described more fully with reference to the accompanying drawings to clarify aspects, other aspects, features and advantages of the present disclosure. The exemplary embodiments may, however, be embodied in many different forms and should not be construed as limited to the exemplary embodiments set forth herein. Rather, the exemplary embodiments are provided so that this disclosure will be thorough and complete, and will fully convey the scope of the application to those of ordinary skill in the art.

It will be understood that when an element is referred to as being "on" (or "under", "on the right of", or "on the left of") another element, the element can be directly on (or "under", "on the right of", or "on the left of") another element or a third element therebetween.

The expressions such as "upper", "lower", "left", "right", "front", "rear", etc. used in the specification to explain a position relationship between first and second elements may be expressions for explaining a relative position of the second element to the first element.

In the detailed description, when a certain element is referred to as being connected (or coupled, secured, attached) to another element, the element may be directly connected (or coupled, secured, attached) to another element or may be indirectly connected (or coupled, secured, attached) to another element with a third element interposed therebetween. In the drawings of the specification, lengths, thicknesses, or wideness of elements are exaggerated for easy understanding of technical features, and a relative size of a certain element to another element may vary according to a specific embodiment.

As used herein, the singular forms are intended to include the plural forms as well, unless the context clearly indicates otherwise. It will be further understood that the terms "comprise" and/or "comprising," when used in this specification, do not preclude the presence or addition of one or more other components.

If the terms such as 'first' and 'second' are used to describe elements, these elements should not be limited by such terms. These terms are used for the purpose of distinguishing one element from another element only. The exemplary embodiments include their complementary embodiments.

Hereinafter, exemplary embodiments will be described in greater detail with reference to the accompanying drawings. The matters defined in the description are provided to assist in a comprehensive understanding of the exemplary embodiments. However, it is apparent that the exemplary embodiments can be carried out by those of ordinary skill in the art without those specifically defined matters. In the description of the exemplary embodiment, certain detailed explanations of related art are omitted when it is deemed that they may unnecessarily obscure the essence of the inventive concept.

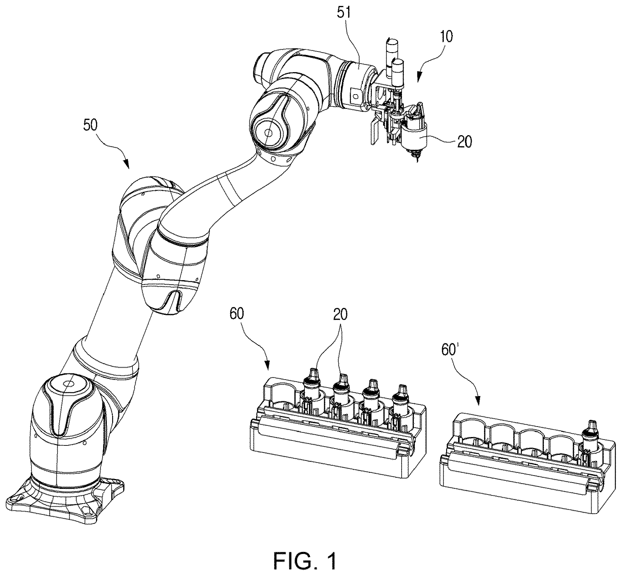

FIG. 1 is a perspective view of an automatic hair transplant device which uses a multi-channel hair transplanter according to an embodiment of the present disclosure. Referring to FIG. 1, the automatic hair transplant device according to an embodiment includes a hair transplanter mounting module 10 (hereinafter, simply referred to as a "mounting module") attached to a robot arm 50, a hair transplanter module 20 removably mounted on the mounting module 10, and one or more hair transplanter stands 60, 60' to hold the hair transplanter module 20. In an embodiment, the automatic hair transplant device may further include a scanning device to 3D model by scanning a treatment area of a patient, a camera to capture a real (visible light) image or an infrared ray image, a laser sensor to measure and trace a distance or position relationship between the mounting module 10 and a treatment area of the patient, and it may be understood that these devices are omitted in FIG. 1.

The robot arm 50 is a device that includes a plurality of joints, a motor to move each joint, and an encoder to detect a motion, and is configured to precisely move a robot arm end 51 by 6 or more degrees of freedom, which is well known in the related-art technology. In the present disclosure, the hair transplanter mounting module 10 may be mounted and installed at the end 51 of the well-known robot arm 50.

In an embodiment, the one or more stands include a first stand 60 and a second stand 60'. The first stand 60 and the second stand 60' have the same shape and structure, but may be distinguished from each other in their purpose. Specifically, the first stand 60 holds one or more multi-channel hair transplanter modules 20 before they are used for a hair transplant procedure, and the second stand 60' holds one or more multi-channel hair transplanter modules 20 after they are used for the hair transplant procedure. However, this is merely an example, and in an alternative embodiment, one stand may hold all of the hair transplanter modules 20 before/after they are used, and three or more stands may be used.

In an embodiment, the hair transplanter mounting module 10 may grip and mount the hair transplanter module 20 held in the first stand 60, and may move the hair transplanter module 20 in a vertical direction, or may perform a hair transplant operation of pressing an upper cap of the hair transplanter module 20 and transplanting hair in a needle of the hair transplanter into patient's scalp. In addition, when the hair transplant operation is completed by the hair transplanter module 20 mounted on the hair transplanter mounting module 10, the mounting module 10 is moved above the second stand 60' by the robot arm 50 and the hair transplanter module 20 is held on the second stand 60'.

The mounting operation, the hair transplant operation, and the placing operation of the hair transplanter mounting module 10 may be performed by a controller (not shown) for controlling the robot arm 50 and the hair transplanter mounting module 10, and it may be understood that the controller is implemented by a computer algorithm, a program, and/or software stored in and executed by a computer device like well-known technology.

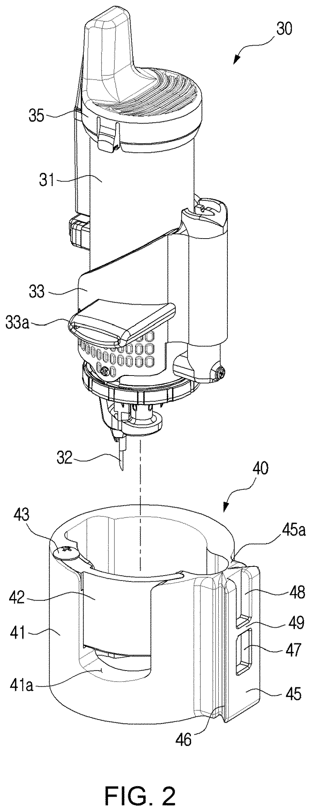

FIG. 2 is a view illustrating a multi-channel hair transplanter and a holder supporting the same according to an embodiment. Referring to FIGS. 1 and 2, the hair transplanter module 20 used in the automatic hair transplant device of the present disclosure may include a multi-channel hair transplanter 30 (hereinafter, simply referred to as a "hair transplanter") and a hair transplanter holder 40 to accommodate and support the hair transplanter 20.

The hair transplanter 30 is a device used for a hair implant, and for example, the hair transplanter 30 may be a multi-channel hair implanter disclosed in Korean Patent Publication No. 2019-0109321 or a hair transplanter having a configuration similar thereto.

In an embodiment, the hair transplanter 30 includes a cylindrical main body 31, an upper cap 35 formed on an upper portion of the main body 31, a sliding handle 33 disposed to surround an outer circumference of the main body 31 at least in part and slidable in the vertical direction with respect to the main body 31, and a plurality of needles 32 arranged around the center axis of the main body 31 in a radial direction inside the main body 31 and protruding to a lower end of the main body 31 one by one in sequence.

In an embodiment, a hand locking portion 33a protrudes from one side surface of the sliding handle 33 of the hair transplanter 30. The hand locking portion 33a protrudes outward in the radial direction in order to prevent the handle 33 from slipping out of user's hand when the user holds and moves up and down the sliding handle 33, and may enhance a grip of the handle 33 and may prevent slip.

When an operator manually performs a hair transplant operation by using the hair transplanter 30 described above, the operator may hold the sliding handle 33 of the hair transplanter 30 with user's thumb and middle finger, and may insert the needle 32 of the hair transplanter into patient's scalp. Thereafter, the operator presses the upper cap 35 with his/her index finger, and moves up the sliding handle 33, and in this process, a hair follicle in the needle 32 is inserted and transplanted into the patient's scalp. Thereafter, the operator moves up the hair transplanter 30 and completely takes out the needle 32 from the patient's scalp, and repeats the above-described operation with respect to a next treatment area.

In order to automatically perform the hair transplant operation of the hair transplanter 30 by using the robot arm 50 and the hair transplanter mounting module 10, a plurality of driving means (for example, a motor or an actuator) for performing the operation of moving up and down the hair transplanter 30, the operation of gripping and moving up and down the sliding handle 33, and the operation of pressing the upper cap 35 should be provided, and operation ranges thereof and timings should be appropriately controlled.

In an embodiment, the hair transplanter holder 40 may be used to hold the hair transplanter 30. The hair transplanter holder 40 may include a substantially cylindrical main body 41 and a side surface protrusion 45 protruding from one side surface of the main body 41 in the radial direction. The main body 41 has a cylindrical shape to surround the outer circumference of the sliding handle 33 of the hair transplanter 30 at least in part. In the illustrated embodiment, the hair transplanter holder 40 may have a penetrating hole 41a formed on one side surface of the main body 41 to accommodate the hand locking portion 33a, and a side surface cover 42 may be secured by a securing means 43 like a bolt or a clip to cover the penetrating hole 41a in part. Accordingly, the side surface cover 42 is removed from the hair transplanter holder 40, first, and the hair transplanter 30 is inserted into the hair transplanter holder 40, such that the hand locking portion 33a of the hair transplanter 30 is seated in the penetrating hole 41a, and the side surface cover 42 is inserted into the hair transplanter holder 40 again and then is secured by the securing means 43, such that the hair transplanter module 20 having the hair transplanter 30 and the hair transplanter holder 40 integrated thereinto is assembled.

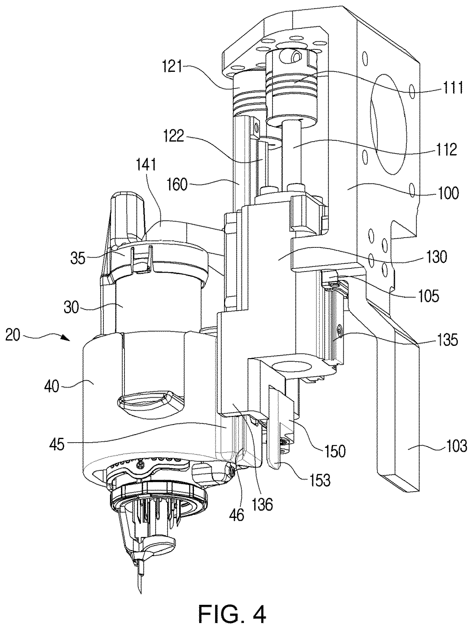

In the illustrated embodiment, an inner rail 46 may be formed on both edges of the side surface protrusion 45 of the hair transplanter holder 40. The inner rail 46 is a member that is engaged with an outer rail 136 of a first sliding block (130 of FIG. 4), which will be described below, to secure the hair transplanter holder 40 to the first sliding block 130. The inner rail 46 may be a concavo-convex structure that is formed on both edges of the side surface protrusion 45 in a longitudinal direction (vertical direction).

The inner rail-outer rail structure is merely an example, and for example, the inner rail may be formed on the first sliding block 130 and the outer rail may be formed on the hair transplanter holder 40. In addition, other well-known structures such as an LM guide may be used to allow the first sliding block 130 to guide the hair transplanter holder 40.

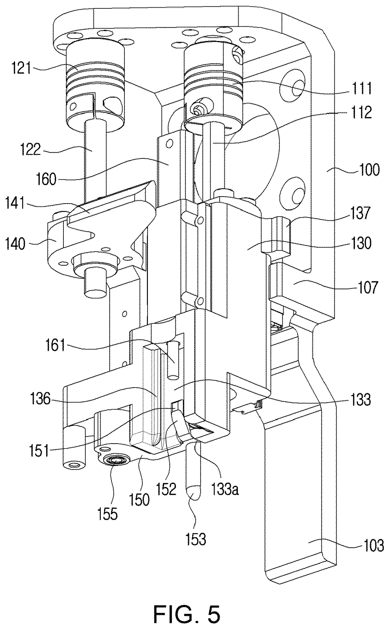

In an embodiment, a first recess 47, a second recess 48, and a step 49 may be formed on the surface of the side surface protrusion 45. The first recess 47 is a concave area that is formed on the surface of the side surface protrusion 45 to a predetermined depth, and the second recess 48 is a concave area that is formed above the first recess 47 and has an upper portion opened. The step 49 is positioned between the first recess 47 and the second recess 48, and has a surface higher than the concave surfaces of the first and second recesses 47, 48. As will be described below, a holder locking protrusion (151 of FIG. 5) of the first sliding block 130 is inserted into the first recess 47, such that the hair transplanter holder 40 is mounted on the first sliding block 130. In addition, since the outer rail 136 of the first sliding block 130 and the inner rail 46 of the hair transplanter holder 40 are engaged with each other and come into close contact with each other, when the hair transplanter holder 40 is mounted on the first sliding block 130, the hair transplanter holder 40 does not shake in the vertical direction or horizontal direction with respect to the first sliding block 130 and is securely fixed.

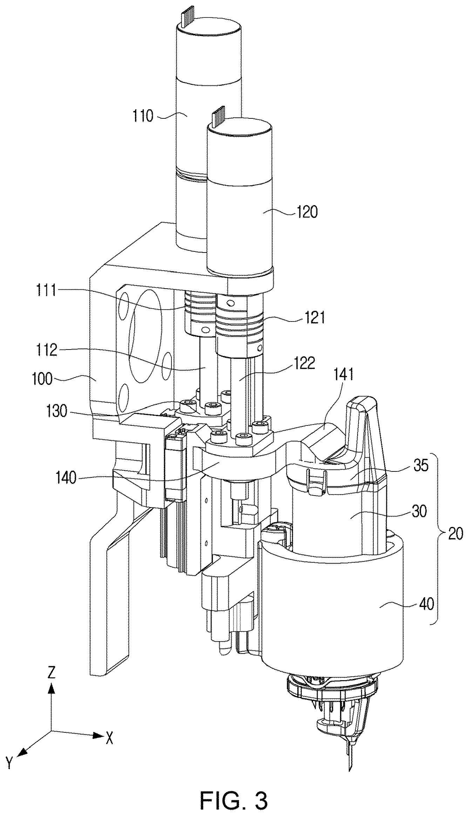

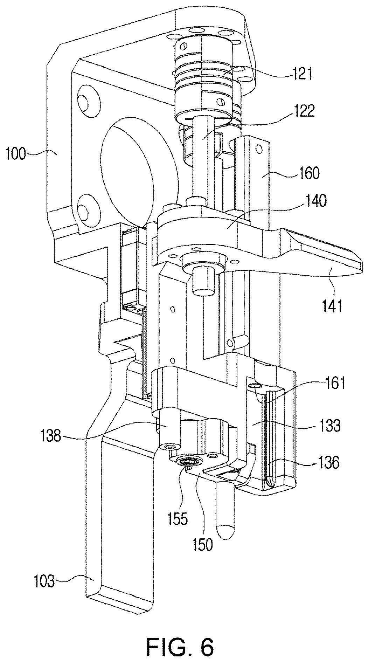

Hereinafter, the hair transplanter mounting module 10 according to an embodiment will be described with reference to FIGS. 3 to 6. FIGS. 3 and 4 are perspective views of the hair transplanter mounding module 10 having the hair transplanter module 20 mounted thereon from different angles, respectively, and FIGS. 5 and 6 are perspective views of the hair transplanter mounting module 10 from different angles when the hair transplanter module 20 is not mounted.

Referring to FIGS. 3 to 6, the hair transplanter mounting module 10 according to an embodiment may include a bracket 100, a first driving unit 110, a second driving unit 120, a first sliding block 130, a second sliding block 140, and a rotation member 150.

The bracket 100 is a member that forms a frame of the hair transplanter mounting module 10, and serves to fix or support various components such as the first and second driving units 110, 120, the first and second sliding blocks 130, 140. The bracket 100 may be removably connected to the end 51 of the robot arm 50.

Each of the first driving unit 110 and the second driving unit 120 may be a driving source for generating a rotation operation or a linear reciprocating operation like an electric motor or an actuator. In the illustrated embodiment, each of the first and second driving units 110, 120 may be implemented by an electric motor, and may be fixedly installed on the bracket 100.

A driving shaft of the first driving unit 110 may be connected with a gear portion 111, and the gear portion 111 may be connected with a shaft 112 disposed on a lower portion thereof, and accordingly, the shaft 112 may be rotated by the first driving unit 110. The first sliding block 130 is coupled to the shaft 112 to be slidable in the vertical direction. For example, the shaft 112 may be a screw shaft that has a screw thread formed on a surface thereof, and the first sliding block 130 may have a screw hole through which the shaft 112 passes, and, when the shaft 112 rotates, the first sliding block 130 may move in the vertical direction. The coupling method of the shaft 112 and the first sliding block 130 by the screw structure described above is merely an example, and the first sliding block 130 may be slidably coupled to the shaft 112 by other well-known coupling methods.

The second driving unit 120 is disposed in parallel with the first driving unit 110 adjacent to the first driving unit 110. A driving shaft of the second driving unit 120 may be connected with a gear portion 121, and the gear portion 121 may be connected with a shaft 122 disposed on a lower portion thereof, and accordingly, the shaft 122 may be rotated by the second driving unit 120. The second sliding block 140 is coupled to the shaft 122 to be slidable in the vertical direction. The coupling structure of the shaft 122 and the second sliding block 140 may be the same as the coupling structure of the shaft 112 and the first sliding block 130 or the shaft 122 and the second slicing block 140 may be slidably coupled to each other by other well-known coupling methods.

The first sliding block 130 may move in the vertical direction with respect to the bracket 100 by the first driving unit 110. Regarding a movement direction of each component in the present disclosure, an X-axis direction refers to a forward and backward direction with reference to the coordinates system shown in FIG. 3, a Y-axis direction refers to a horizontal direction, and a Z-axis direction refers to a vertical direction for convenience of explanation unless a movement direction is specified for a specific component.

In an embodiment, in order to couple the first sliding block 130 to the bracket 100 slidably and securely, the bracket 100 and the first sliding block 130 may be configured to be engaged with each other via a guide rail. For example, as shown in FIG. 4, an outer rail 105 in the vertical direction may be formed on a surface of the bracket 100 that faces the first sliding block 130, and an inner rail 135 in the vertical direction may be formed on a surface of the first sliding block 130 that faces the bracket 100, and the outer rail 105 and the inner rail 135 may be engaged with each other and may slide. Accordingly, the first sliding block 130 may move in the vertical direction by the first driving unit 100, while being securely coupled to the bracket 100 due to the outer rail-inner rail structure, so that the first sliding block 130 is prevented from shaking when moving.

The inner rail-outer rail structure described above is merely an example. For example, the inner rail may be formed on the bracket 100 and the outer rail may be formed on the first sliding block 130, and in addition, the bracket 100 may guide the first sliding block 130 by using other well-known structures such as an LM guide, etc.

In an embodiment, the hair transplanter module 20 is removably mounted on the first sliding block 130. Referring to FIGS. 5 and 6, a holder insertion portion 133 is formed on a surface of the first sliding block 130 that faces the hair transplanter module 20 to have the side surface protrusion 45 of the hair transplanter holder 40 inserted thereinto. The holder insertion portion 133 may be formed as a concave recess on a surface of the first sliding block 130 as shown in the drawing, and may have a lower portion opened, such that the side surface protrusion 45 of the hair transplanter holder 40 slides upward from a lower portion of the holder insertion portion 133 and is inserted into the holder insertion portion 133.

The outer rail 136 may be formed on both left and right sides of the holder insertion portion 133, and, in response to this, the inner rail 46 may be formed on both edges of the side surface protrusion 45 of the hair transplanter holder 40 to be engaged and coupled with the outer rail 136. Accordingly, when the side surface protrusion 45 of the hair transplanter holder 40 moves upward from the lower portion of the holder insertion portion 133, the inner rail 46 and the outer rail 136 are engaged with and fitted into each other, and in this state, the side surface protrusion 45 further moves up and is completely inserted into the holder insertion portion 133.

The rotation member 150 may be attached to a lower end of the first sliding block 130. As shown in FIGS. 5 and 6, the rotation member 150 may have a rotation shaft 155 formed at one end therein in the horizontal direction, and a holder locking protrusion 151 formed at the other end. The holder locking protrusion 151 may protrude in a horizontally forward direction and may have a size and a shape to be inserted into the first recess 47 of the hair transplanter holder 40. In addition, the holder locking protrusion 151 may have an inclined surface 152 formed on a lower surface thereof.

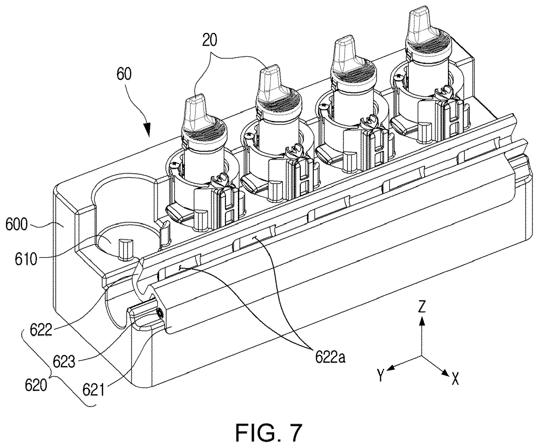

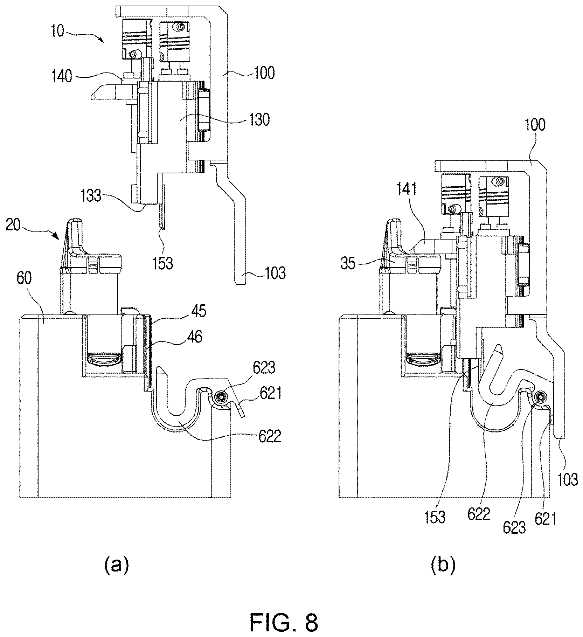

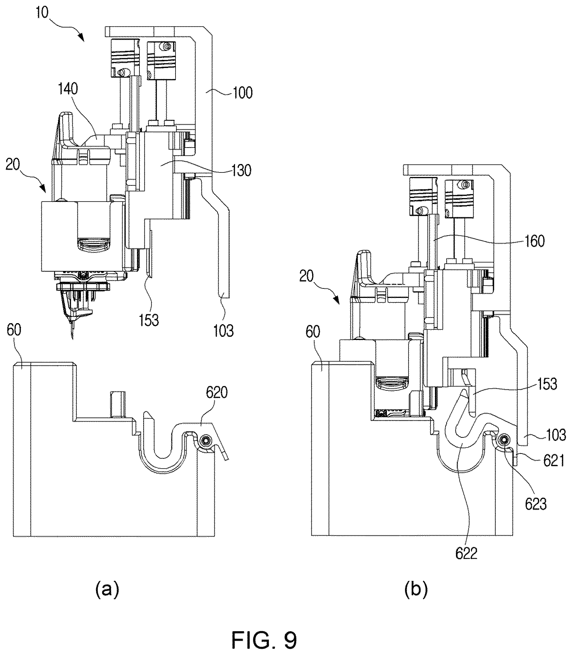

In an embodiment, the rotation member 150 may further include a lever locking portion 153 extended downward therefrom. The lever locking portion 153 may be locked into a lever (620 of FIG. 7) of the stand 60, thereby rotating the rotation member 150, and accordingly, the rotation member 150 may switch from a closed state to an open state. Operations of the lever locking portion 153 and the lever 620 will be described below with reference to FIGS. 8 and 9.

The rotation member 150 may switch between the "closed state" and the "open state" by rotation. The "closed state" is illustrated in FIG. 5 or 6, and refers to a state where the locking protrusion 151 of the rotation member 150 passes through a slot 133a of the holder insertion portion 133 and protrudes further forward than the surface of the holder insertion portion 133 (that is, toward the hair transplanter holder 40). The "open state" refers to a state where the rotation member 150 rotates backward (that is, in a direction of going away from the hair transplanter holder 40) and the holder locking protrusion 151 is released from the slot 133a of the holder insertion portion 133. In an embodiment, when a force is not applied to the rotation member 150 from the outside, the rotation member 150 is configured to maintain the closed state. For example, the rotation member 150 may be configured to be in the closed state by using an elastic means such as a spring when an external force is not applied.

According to the above-described configuration, when the hair transplanter holder 40 slides upward with the inner rail 46 of the side surface protrusion 45 of the hair transplanter holder 40 and the outer rail 136 of the holder insertion portion 133 being engaged with each other, the holder locking protrusion 151 in the "closed state" relatively moves downward from an upper portion of the side surface protrusion 45 of the hair transplanter holder 40, and the holder locking protrusion 151 passes through the second recess 48 and the step 49 and is inserted into and seated in the first recess 47, such that the hair transplanter holder 40 is mounted on the first sliding block 130. In this state, the holder locking protrusion 151 is inserted into the first recess 47 and the side surface protrusion 45 of the hair transplanter holder 40 is inserted into the holder insertion portion 133 by the inner rail-outer rail structure on both sides. Accordingly, the hair transplanter holder 40 may be securely mounted on the first sliding block 130 without being shaken in the horizontal direction or in the vertical direction.

In the illustrated embodiment, the first sliding block 130 may further include a push bar 160 that moves up and down. The push bar 160 is a bar-shaped member and is positioned on a front surface of the first sliding block 130, and preferably, as shown in FIG. 5, a lower end 161 of the push bar 160 may move down toward a space of the holder insertion portion 133.

The push bar 160 is coupled to the first sliding block 130 by an elastic member such as a spring, etc., and, when no force is applied to the push bar 160 from the outside, the push bar 160 may slide downward and may have the end 161 protrude toward the space of the holder insertion portion 133 as shown in FIG. 5.

Referring to FIG. 5, the first sliding block 130 may further include an extension portion 137 extended therefrom in a lateral direction, and in response to this, the bracket 100 may further include a stopper 107 protruding forward therefrom. The extension portion 137 and the stopper 107 are positioned on positions to interfere with each other. Accordingly, when the first sliding block 130 moves downward by a predetermined distance or longer, the extension portion 137 collides with the stopper 107, such that the downward movement of the first sliding block 130 is restricted.

The second sliding block 140 is disposed on one side of the first sliding block 130 in parallel, and is configured to move in the vertical direction with respect to the bracket 100 by the second driving unit 120. The second sliding block 140 is disposed adjacent to the first sliding block 130. The second sliding block 130 includes a cap pressing portion 141 protruding forward therefrom.

When the hair transplanter module 20 is mounted on the first sliding block 130 as shown in FIGS. 3 and 4, the cap pressing portion 141 of he second sliding block 140 comes into close contact with the upper cap 35 of the hair transplanter 30, and, when the second sliding block 140 moves downward, the cap pressing portion 141 presses the cap 35.

FIG. 7 is a perspective view of the hair transplanter stand 60 according to an embodiment. Since the first stand 60 shown in FIG. 1 and the second stand 60' have the same structure, only the first stand 60 will be described hereinbelow and the "first stand" may be referred to as simply a "stand".

Referring to FIG. 7, the hair transplanter stand 60 includes a stand block 600 provided with a plurality of hair transplanter seating portions 610. As shown in FIG. 7, the plurality of seating portions 610 may be arranged in a line, and one hair transplanter module 20 may be accommodated in every hair transplanter seating portion 610.

On the assumption that the X-axis direction of FIG. 7 is a forward and backward direction, a lever 620 is installed on a front portion of the hair transplanter seating portions 610 of the stand block 600. The lever 620 may have a length over the entire hair transplanter seating portions 610 in a longitudinal direction (that is, the Y-axis direction) of the stand block 600. In an alternative embodiment, individual levers 620 may be independently installed on a front portion of every seating portions 610.

In an embodiment, when the hair transplanter mounting module 10 having the multi-channel hair transplanter module 20 mounted thereon moves down from above the stand 60, the hair transplanter mounting module 10 presses one end of the lever 620 and the multi-channel hair transplanter module 20 is configured to be dismounted from the first sliding block 130 by the lever 620.

The lever 620 includes a first rotation portion 621 and a second rotation portion 622 which move in the vertical direction (that is, the Z-axis direction) with reference to a lever shaft 623 disposed in the longitudinal direction of the stand block 600. The first rotation portion 621 is positioned on a front portion of the lever shaft 623, and the second rotation portion 622 is positioned on a rear portion of the lever shaft 623. In the illustrated embodiment, the second rotation portion 622 may have penetrating holes 622a formed on areas facing the first recesses 47 of the hair transplanters 20 held in the seating portions 610. The penetrating hole 622a is formed to prevent the lever locking portion 153 and the second rotation portion 622 from interfering with each other when a mounting operation is performed, which will be described below with reference to FIG. 8.

Hereinafter, an operation of mounting the hair transplanter module 20 held on the stand 60 on the mounting module 10, and an operation of placing the hair transplanter module 20 on the stand 60 will be described with reference to FIGS. 8 and 9.

First, FIG. 8 illustrates the operation of mounting the hair transplanter module 20 held on the hair transplanter stand 60 on the mounting module 10.

Referring to FIG. 8A, the hair transplanter module 20 is held on the stand 60 and the mounting module 10 is positioned above the stand 60. In this case, the inner rail 46 of the side surface protrusion 45 of the hair transplanter holder 40 and the outer rail 136 of the first sliding block 130 are aligned with each other in the vertical direction. In addition, in FIG. 8A, the first sliding block 130 and the second sliding block 140 are moved upward and are in a relatively higher position with respect to the bracket 100, and accordingly, the bracket lower end 103 is relatively lower than the lever locking portion 153.

In this arrangement state, the mounting module 10 moves down toward the stand 60 by an operation of the robot arm 50. When the mounting module 10 moves down, the lower end 103 of the bracket presses the first rotation portion 621 of the lever 620 and the second rotation portion 622 moves up and is maintained in an elevated position. When the mounting module 10 continues moving down, the inner rail 46 of the hair transplanter holder 40 and the outer rail 136 of the first sliding block 130 are engaged with each other, and the holder locking protrusion 151 of the rotation member 150 in the closed state goes through the second recess 48 and the step 49 of the hair transplanter holder 40 and moves down, and is inserted into and seated in the first recess 47, such that the hair transplanter module 20 is mounted on the mounting module 10.

In this case, while the lever locking protrusion 151 moves down and is inserted into the first recess 47, the second rotation portion 622 of the lever 620 is in the elevated position as shown in FIG. 8B, and, since the second rotation portion 622 has the penetrating hole 622a formed thereon, the lever locking protrusion 151 is not disturbed by the second rotation portion 622 and the rotation member 150 is maintained in the closed state.

When the hair transplanter module 20 is mounted on the mounting module 10 as described above, the hair transplanter module 20 is moved to a treatment area of the patient by moving the robot arm 50, and the controller controls the first sliding block 130 and the second sliding block 140, respectively, to perform a hair transplant operation of the hair transplanter 30.

FIG. 9 illustrates the operation of placing the hair transplanter module 20 mounted on the hair transplanter mounting module 10 on the hair transplanter stand 60.

Referring to FIG. 9A, the hair transplanter module 20 is mounted on the mounting module 10 and the mounting module 10 is positioned above the empty hair transplanter seating portion 610 of the stand 60. Compared to FIG. 8A, FIG. 9A illustrates that the first sliding block 130 is moved downward and is in a relatively lower position with respect to the bracket 100, and accordingly, the lower end 103 of the bracket and the lever locking portion 153 are positioned at similar height.

In this arrangement state, the mounting module 10 moves down toward the stand 60 by an operation of the robot arm 50. When the mounting module 10 moves down, the lower end 103 of the bracket presses the first rotation portion 621 of the lever 620 as shown in FIG. 9B, and the second rotation 622 moves up. In this case, the level locking portion 153 is locked by the second rotation portion 622 and the rotation member 150 is rotated, such that the rotation member 150 switches into the open state. Accordingly, the holder locking protrusion 151 of the rotation member 150 is released from the first recess 47 of the hair transplanter holder 40, and the hair transplanter module 20 moves down due to its weight, such that the hair transplanter module 20 is seated in the seating portion 610 of the stand 60.

In an embodiment, when the first sliding block 130 includes the push bar 160, the push bar 160 may assist the downward movement of the hair transplanter module 20. The push bar 160 tends to move downward due to elasticity. Accordingly, even when the hair transplanter holder 40 is mounted on the first sliding block 130, the end 161 of the push bar 160 always presses an upper surface (that is, 45a of FIG. 2) of the side surface protrusion 45 of the hair transplanter holder 40. When the rotation member 150 switches into the open state and the holder locking protrusion 151 is released from the first recess 47 of the hair transplanter holder 40, it is assured that the hair transplanter module 20 slides down below the first sliding block 130 and is released therefrom not only due to the weight of the hair transplanter module 20 but also due to the pressing operation of the push bar end 161 of pushing down the hair transplanter holder 40.

When the hair transplanter module 20 is held on the stand 60 as described above, the mounting module 10 is moved above the hair transplanter module 20 held on another seating portion 610 of the stand 60 by moving the robot arm 50, and the operation of mounting the hair transplanter module 20 may be performed as shown in FIG. 8. Accordingly, the operations of replacing the hair transplanter module 20 and transplanting hair into the patient may be automatically performed without intervention by an operator.

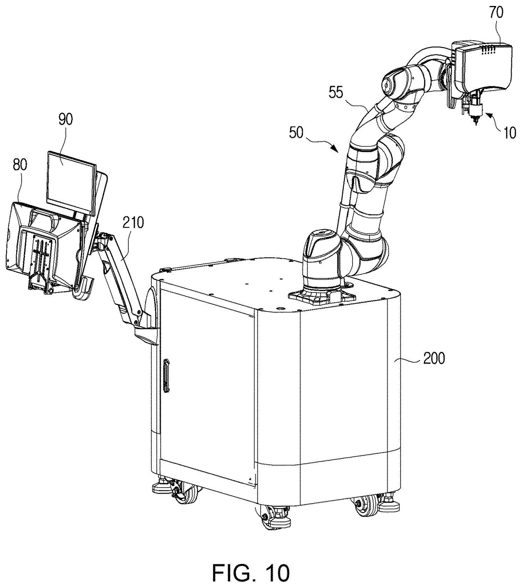

An exemplary configuration of an automatic hair transplant device system according to an embodiment will be described hereinbelow with reference to FIGS. 10 to 13.

FIG. 10 schematically illustrates an exemplary configuration of an entire automatic hair transplant device system provided with a robot arm 50 and a hair transplanter mounting module 10 according to an embodiment. Referring to FIG. 10, the system according to an embodiment may include a main body 200, the robot arm 50 installed on the main body 200, the hair transplanter module 10 and a 3D scanner 70 which are removably mounted on the robot arm 50, and a keyboard 80 and a display 90 which are installed on a holder 210 of the main body 200. In an embodiment, in addition to the keyboard 80, other input devices such as a mouse (not shown) may be additionally placed on the holder 210 and may be used. It will be understood that the present disclosure is not limited to a specific input device such as the keyboard 80, the mouse, etc.

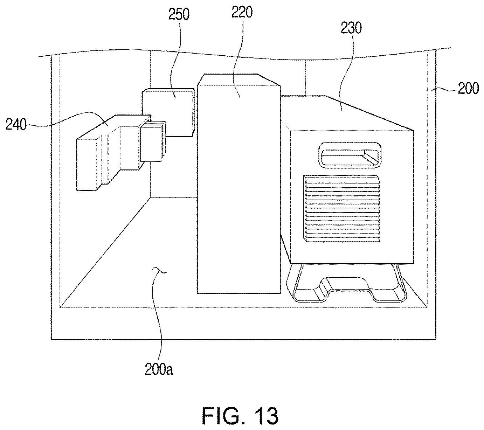

The main body 200 may be provided with various computer devices, a control device, and a power supply device, which will be described below with reference to FIG. 13, and the robot arm 50 may be installed on an upper portion of the main body 200 and the hair transplanter mounting module 10 may be attached to a leading end of the robot arm 50. The robot arm 50 and the hair transplanter mounting module 10 may be the same or similar devices as or to those described above with reference to FIGS. 1 to 9.

The main body 200 may include the holder 210 of a foldable structure, and the key board 80 and the display 90 may be mounted on an end of the holder 210. A user may input a user command through an input/output device such as the keyboard 80 or the display 90 in order to operate the automatic hair transplant device system, and may control an operation of a hair transplant device.

The 3D scanner 70 may be installed on an end of the robot arm 50 adjacent to the hair transplanter mounting module 10. The 3D scanner 70 may 3D model by scanning a treatment area of a patient. In an alternative embodiment, a device such as a camera for capturing a real (visible light) image or an infrared ray image and/or a laser sensor for measuring and tracing a distance or a position relationship between the hair transplanter mounting module 10 and a treatment area of a patient may further be provided in the 3D scanner 70.

In the illustrated embodiment, a hose 55 may be installed in parallel with the robot arm 50, and various electric wires may pass through the hose 55. For example, when a control line or a power line should be connected to the hair transplanter mounting module 10 and/or the 3D scanner 70 from the main body 200, the control line or the power line may be extended from the main body 200 through the hose 55 and may be connected to the hair transplanter mounting module 10 and/or the 3D scanner 70.

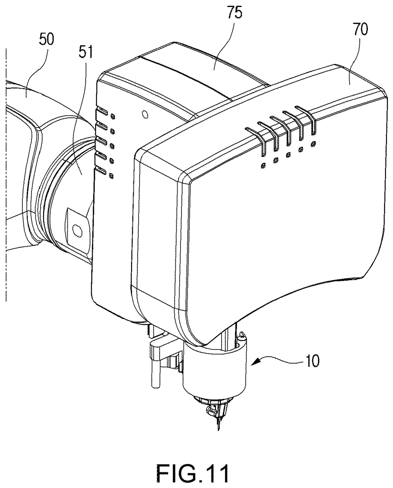

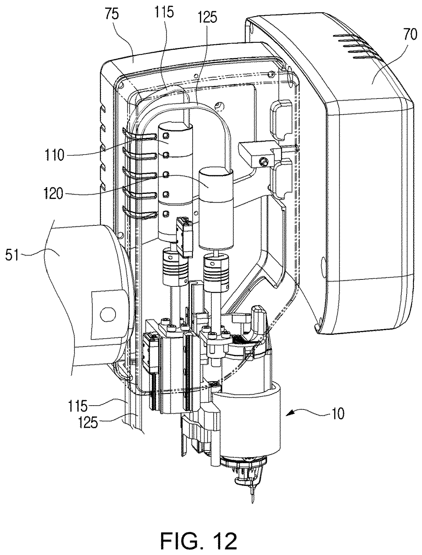

FIG. 11 is an enlarged view of the hair transplanter mounting module 10 and the 3D scanner 70 mounted on the end of the robot arm 50. FIG. 12 is a view schematically illustrating an interior structure of a cover member 75.

Referring to FIGS. 11 and 12, the hair transplanter mounting module 10 may be attached to the leading end 51 of the robot arm 50, and the cover member 75 may be attached to the leading end 51 of the robot arm 50. The cover member 75 has a shape to surround an upper portion and a side surface of the hair transplanter mounting module 10 at least in part. In an embodiment, the 3D scanner 70 may be installed on a front surface of the cover member 75.

Although not shown in FIGS. 11 and 12, a hose (55 of FIG. 10) may be extended to the leading end 51 of the robot arm 50. Accordingly, control lines 115, 125 extended from the first and second driving units 110, 120 of the hair transplanter mounting module 10 may enter one end of the hose 55, pass through the inside of the hose 55, and may be connected to the main body 200.

FIG. 13 schematically illustrates exemplary devices installed in the main body 200. In FIG. 13, the main body 200 has an inner space 200a for installing devices therein. In the illustrated embodiment, a computer main body 220, a robot arm control device 230, a driving unit control device 240, and a power supply device 250 may be installed in the inner space 200a.

The computer main body 220 may be a typical PC computer main body and may be used to execute a program for controlling the overall system. For example, the computer main body 220 may be wiredly or wirelessly connected with the robot arm control device 230, the driving unit control device 240, the 3D scanner 70, the keyboard 80, and the display 90.

The robot arm control device 230 is a device for moving the robot arm 50 by controlling the robot arm 50, and for example, may receive a command to operate the robot arm 50 according to an automatic hair transplant program executed in the computer main body 220, and may operate a plurality of motors installed in the robot arm 50 according to the command and may control the robot arm 50 to perform a specific operation.

The driving unit control device 240 is a device for controlling the first and second driving units 110, 120 of the hair transplanter mounting module 10, and for example, may be connected with the control lines 115, 125 described with reference to FIG. 11. For example, the driving unit control device 240 may receive a command to operate the first and second driving units 110, 120 according to the automatic hair transplant program executed in the computer main body 220, and may control the first and second driving units 110, 120 to operate according to the command.

The power supply device 250 is a device for supplying power supplied from the outside to various devices such as the computer main body 220, the control devices 230, 240, the robot arm 50, the hair transplanter mounting module 10, and for example, may be implemented by an SMPS.

Various modifications and changes can be made by those skilled in the art based on the above descriptions of the disclosure. Therefore, the scope of the present disclosure is defined not by the detailed description of the embodiments but by the appended claims and equivalents thereto.

## Claims

1. A hair transplanter mounting module which is mounted on a robot arm configured with a plurality of arms and a plurality of joints, the hair transplanter mounting module comprising:
a bracket 100 which is coupled to the robot arm;
a first sliding block 130 which is installed on the bracket and is slidable in a vertical direction with respect to the bracket; and
a second sliding block 140 which is installed on the bracket and is disposed at one side of the first sliding block in parallel, and is slidable in the vertical direction with respect to the bracket,
wherein a multi-channel hair transplanter module 20 is removably mounted on the first sliding block 130.

2. The hair transplanter mounting module of claim 1, comprising:
a first rail 105 which is formed on the bracket by a predetermined length in the vertical direction; and
a second rail 135 which is formed on the first sliding block 130 by a predetermined length in the vertical direction, and is engaged with and slidably coupled with the first rail.

3. The hair transplanter mounting module of claim 1, wherein the multi-channel hair transplanter module 20 comprises:
a multi-channel hair transplanter 30 provided with a plurality of needles which are arranged around a center axis in a radial direction; and
a hair transplanter holder 40 which is removably coupled to a sliding handle 33 on an outer circumference of the multi-channel hair transplanter 30.

4. The hair transplanter mounting module of claim 3, wherein a third rail 136 is formed on the first sliding block 130 by a predetermined length in the vertical direction, and
wherein the hair transplanter holder 40 comprises a fourth rail 46 which is formed on one side surface of the hair transplanter holder 40 and is engaged with the third rail and is coupled to be slidable in the vertical direction.

5. The hair transplanter mounting module of claim 4, wherein the first sliding block 130 further comprises a holder locking protrusion 151 formed on a surface of the first sliding block that faces the hair transplanter holder,
wherein the hair transplanter holder 40 further comprises a first recess 47 which is formed on a surface of the hair transplanter holder that faces the first sliding block, and accommodates the holder locking protrusion 151, and
wherein the holder locking protrusion 151 is inserted into the first recess 47 with the third rail 136 of the first sliding block and the fourth rail 46 of the hair transplanter holder being engaged with each other, such that the multi-channel hair transplanter module 20 is mounted on the first sliding block 130.

6. The hair transplanter mounting module of claim 4, further comprising a bush bar 160 which is installed on an upper portion of the third rail 136 of the first sliding block 130 and is configured to press down the hair transplanter holder 40 mounted on the first sliding block 130 by using an elastic force.

7. The hair transplanter mounting module of claim 1, wherein, when the multi-channel hair transplanter module 20 is mounted on the first sliding block 130, the second sliding block 140 is configured to press an upper end of the multi-channel hair transplanter module.

8. An automatic hair transplant device which automatically performs a hair transplant operation of transplanting hair into patient's scalp by using a hair transplanter, the automatic hair transplant device comprising:
a hair transplanter mounting module 10 attached to a multi-joint robot arm 50 according to any one of claims 1 to 7;
one or more stands configured to hold a multi-channel hair transplanter module 20 according to any one of claims 1 to 7; and
a controller configured to control operations of the robot arm and the hair transplanter mounting module,
wherein the robot arm and the hair transplanter mounting module are operated under control of the controller to perform an operation of mounting the multi-channel hair transplanter module 20 held on the stand on the hair transplanter mounting module 10, and an operation of placing the multi-channel hair transplanter module 20 mounted on the hair transplanter mounting module 10 on the stand 60.

9. The automatic hair transplant device of claim 8, wherein the stand comprises a plurality of hair transplanter seating portions 610 configured to accommodate the multi-channel hair transplanter modules, respectively, and a lever 620 installed adjacent to the hair transplanter seating portions, and
wherein, when the hair transplanter mounting module 10 having the multi-channel hair transplanter module mounted thereon moves down, the hair transplanter mounting module presses one end of the lever 620 and the multi-channel hair transplanter module is dismounted from the first sliding block 130 by the lever.

10. The automatic hair transplant device of claim 8, wherein the one or more stands comprise a first stand 60 and a second stand 60', and
wherein the first stand is configured to hold one or more multi-channel hair transplanter modules before the multi-channel hair transplanter modules are used for a hair transplant procedure, and the second stand is configured to hold one or more multi-channel hair transplanter modules which are used for the hair transplant procedure.
